# EUROPÄISCHE PATENTSCHRIFT

(11) **EP 1 082 317 B1**
(45) Veröffentlichungstag und Bekanntmachung des Hinweises auf die Patenterteilung: **05.03.2003**
(21) Anmeldenummer: 99924921.2
(22) Anmeldetag: 10.05.1999
(51) Int. Cl.: C07D 307/89, C07C 51/31, C07C 51/265, C07C 63/16

(54) **VERFAHREN ZUR HERSTELLUNG VON PHTHALSÄUREANHYDRID DURCH KATALYTISCHE GASPHASENOXIDATION VON O-XYLOL/NAPHTHALIN-GEMISCHEN**
METHOD FOR PRODUCING PHTHALIC ANHYDRIDE BY MEANS OF CATALYTIC VAPOR-PHASE OXIDATION OF O-XYLOL/NAPHTHALENE MIXTURES
PROCEDE DE PRODUCTION D'ANHYDRIDE PHTALIQUE PAR OXYDATION CATALYTIQUE EN PHASE GAZEUSE DE MELANGES O-XYLENE/NAPHTALENE

(30) Priorität: 26.05.1998 DE 19823275
(43) Veröffentlichungstag der Anmeldung: 14.03.2001
(73) Patentinhaber: BASF AKTIENGESELLSCHAFT, 67056 Ludwigshafen (DE)
(72) Erfinder: HEIDEMANN, Thomas, D-69469 Weinheim (DE); ARNOLD, Heiko, D-68159 Mannheim (DE); HEFELE, Gerhard, D-67354 Römerberg (DE); WANJEK, Herbert, D-67133 Maxdorf (DE)
(86) Internationale Anmeldenummer: EP9903192
(87) Internationale Veröffentlichungsnummer: WO99061434

(56) Entgegenhaltungen:
- EP-A- 0 286 448
- EP-A- 0 539 878
- US-A- 4 046 780

## Beschreibung

Die Erfindung betrifft ein Verfahren zur Herstellung von Phthalsäureanhydrid durch katalytische Gasphasenoxidation von o-Xylol/Naphthalin-Gemischen mit molekularem Sauerstoff unter Verwendung von 2 Katalysatorbetten aus Schalenkatalysatoren, die Cäsium enthalten, wobei der Cäsiumgehalt des Katalysators der zweiten Schicht weniger als 15 Gew.-% des Cäsiumgehalts der ersten Schicht beträgt.

Die Gasphasenoxidation von o-Xylol und/oder Naphtalin zu Phthalsäureanhydrid ist wohl bekannt und in der Literatur vielfach beschrieben. Dazu wird im Allgemeinen ein Gemisch aus einem molekularen Sauerstoff enthaltenden Gas, beispielsweise Luft, und das zu oxidierende Ausgangsmaterial durch eine Vielzahl in einem Reaktor angeordneter Rohre geleitet, in denen sich eine Schüttung mindestens eines Katalysators befindet. Zur Temperaturregelung sind die Rohre von einem Wärmeträgermedium, beispielsweise eine Salzschmelze, umgeben. Trotz dieser Thermostatisierung kann es in der Katalysatorschüttung zur Ausbildung sogenannter "Heißer Flekken" (hot spots) kommen, in denen eine höhere Temperatur herrscht als im übrigen Teil der Katalysatorschüttung. Diese "hot spots" geben Anlaß zu Nebenreaktionen, wie der Totalverbrennung des Ausgangsmaterials, oder sie führen zur Bildung unerwünschter, vom Reaktionsprodukt nicht oder nur mit viel Aufwand abtrennbarer Nebenprodukte, beispielsweise zur Bildung von Phthalid oder Naphthochinon. Zur Abschwächung dieses hot spots wurde bereits vorgeschlagen, unterschiedlich aktive Katalysatoren schichtweise in der Katalysatorschüttung anzuordnen, wobei in der Regel der weniger aktive Katalysator zum Gaseintritt hin, und der aktivere Katalysator zum Gasaustritt aus der Katalysatorschüttung hin angeordnet ist. Die unterschiedlich aktiven Katalysatoren in der Katalysatorschüttung können bei der gleichen Temperatur dem Reaktionsgas ausgesetzt werden, es können die beiden Schichten aus unterschiedlich aktiven Katalysatoren aber auch auf unterschiedliche Reaktionstemperaturen thermostatisch mit dem Reaktionsgas in Kontakt gebracht werden.

Als Katalysatoren für die Phthalsäureanhydridherstellung haben sich sogenannte Schalenkatalysatoren bewährt (s.u.).

Zur Oxidation von Naphthalin oder o-Xylol werden aufgrund der unterschiedlichen Reaktivitäten der Edukte im Allgemeinen verschiedene Katalysatoren eingesetzt, so dass bei dem Einsatz von o-Xylol/Naphthalin-Gemischen speziell angepaßte Katalysatoren eingesetzt werden müssen, die sich sowohl zur o-Xylol- wie auch zur Naphthalinoxidation eignen, wie dies z.B. in BE 893521, EP 286448 sowie in DE 2238067 beschrieben ist.

Gemäß einer speziellen Lösung dieses Problems wird in EP 539878 beschrieben, dass bei Einsatz von Katalysatoren der Eingangsstufe in einer Betthöhe von 15-85%, bezogen auf das Volumen der Gesamthöhe des Katalysatorbettes, und eines Katalysators der darauf folgenden Stufe in einer Betthöhe von 85-15%, bezogen auf das Volumen der Gesamthöhe des Katalysatorbettes, in Richtung Gasausgang, in Form von übereinanderliegenden Schichten, sehr gute Ergebnisse erzielt werden, wenn man beim Katalysator der Eingangsstufe eine Katalysatorsubstanz auf einen inaktiven Träger im Verhältnis im Bereich zwischen 5 und 20g/100ml aufbringt, wobei die katalytische Substanz 1 bis 20 Gew.-% V₂O₅ und 99 bis 80 Gew.-% Titandioxid vom Anatastyp mit einer spezifischen Oberfläche von 10 bis 60 m²/g umfaßt, sowie ferner die Einarbeitung von, bezogen auf 100 Gewichtsteile der Gesamtmenge vorgenannter beider Komponenten, 0,01 bis 1 Gew.-% Nb₂O₅, 0,2 bis 1,2 Gew.-% P₂O₅, 0,5 bis 5 Gew.-% Sb₂O₃ und 0,3 bis 1,2 Gew.-% von zumindest einer Verbindung ausgewählt aus der Gruppe bestehend aus Kalium, Cäsium, Rubidium und Thallium als Oxid aufweist, wobei beim Katalysator der zweiten Schicht die Menge einer Verbindung, ausgewählt aus der Gruppe bestehend aus Kalium, Cäsium, Rubidium und Thallium 17 bis 63 Gew.-% der Menge dieser entsprechenden Verbindung in dem Katalysator der Eingangsstufe beträgt. Außerhalb dieses "Fensters" (Variation des Längenverhältnis des späteren Katalysators zur Gesamtlänge von 85-15% und dem Alkaliverhältnis von 17-63%) ist nach den Angaben dieser Patentschrift die Reaktion "schwierig" oder mit höheren Ausbeuteverlusten verbunden.

Es wurde nun überraschenderweise gefunden, dass Katalysatoren mit nahezu obiger Zusammensetzung unter Einsatz von Cäsium als Alkalimetall und einem Cäsiumgehalt der zweiten Schicht von weniger als 15 Gew.-% des Cäsiumgehalts der ersten Schicht besonders vorteilhafte Eigenschaften im Vergleich zu den obigen Katalysatoren in dem genannten "Fenster" aufweisen.

Im Einzelnen wurde ein verbessertes Verfahren zur Herstellung von Phthalsäureanhydrid gefunden durch katalytische Gasphasenoxidation von o-Xylol/Naphtalingemischen mit molekularem Sauerstoff unter Verwendung eines
Katalysators I in einer ersten Schicht auf der Gaseintrittsseite, die 25 bis 75 Volumenprozent des gesamten Katalysatorvolumens ausmacht, enthaltend, jeweils bezogen auf die katalytisch wirksame Masse, 1 bis 10 Gew.-% Vanadiumoxid (berechnet als V₂O₅), 1 bis 10 Gew.-% Antimonoxid (berechnet als Sb₂O₃) und 80 bis 98 Gew.-% Titandioxid vom Anatas-Typ mit einer BET-Oberfläche von 13 bis 28 m²/g sowie 0,05 bis 1 Gew.-% Cäsium (berechnet als Cs), aufgebracht auf einem Steatitträger und eines

Katalysators II in einer zweiten Schicht, die die restlichen 75 bis 25 Volumenprozent des gesamten Katalysatorvolumens ausmacht, enthaltend, jeweils bezogen auf die katalytisch wirksame Masse, 1 bis 10 Gew.-% Vanadiumoxid (berechnet als V₂O₅), 1 bis 10 Gew.-% Antimonoxid (berechnet als Sb₂O₃) und 80 bis 98 Gew.-% Titanoxid vom Anatas-Typ mit einer BET-Oberfläche (vgl. J. Amer. Chem. Soc. Band 60, Seite 309 ff. (1938)) von 13 bis 28 m²/g und 0,01 bis 1 Gew.-% Phosphoroxid (berechnet als P) sowie 0,01 bis 0,2 Gew.-% Cäsium (berechnet als Cs), aufgebracht auf einem Steatitträger, wobei der Cäsiumgehalt des Katalysators II weniger als 15 Gew.-% des Cäsiumgehalts des Katalysators I beträgt und der Katalysator I und der Katalysator II ohne Zusatz von Verbindungen des Niobs hergestellt sind.

Durch die Abreicherung des Cäsiumgehaltes in dem Katalysator II auf unter 15 Gew.-% des Cäsiumgehaltes des Katalysators I, kann die Produktqualität durch Verringerung des Gehalts an den unerwünschten Nebenprodukten Phthalid bzw. Naphthochinon deutlich erhöht werden ohne signifikante Beeinflussung der Phthalsäureanhydrid-Ausbeute. Dies wirkt sich insbesondere bei tieferen Badtemperaturen aus, die zur Erreichung von Beladungen über 60g/Nm³ notwendig sind. Besonders deutlich tritt dieser Effekt bei einem Cäsiumgehalt des Katalysators II von 10-13 Gew.-% des Cäsiumgehalts von Katalysator I und einem Anteil der Schicht des Katalysators II an der gesamten Katalysatorschüttung von 40-60% auf.

Die verwendeten Katalysatoren können auch ohne Dotierung geringe Mengen an Metalloxiden wie die des Niobs, des Wolframs und/oder des Bleis enthalten. Diese kommen über mögliche Verunreinigungen des verwendeten handelsüblichen Anatas zustande und sind somit nicht als Dotierung von Aktivmassen zu werten. Weitere Verunreinigungen, insbesondere Alkalimetallverunreinigungen, liegen i. Allg. unter 0,01 Gew.-%.

Für das erfindungsgemäße Verfahren kommen im Rahmen der anspruchsgemäßen Grenzen an sich bekannte Schalenkatalysatoren in Betracht, wie sie in der Fachliteratur für die Herstellung von Phthalsäureanhydrid beschrieben sind.Zusammenfassend kann die Zusammensetzung und Herstellung solcher "Standardkatalysatoren" wie folgt beschrieben werden:

Es haben sich in der Regel Schalenkatalysatoren bewährt, bei denen die katalytisch aktive Masse ("Aktivmasse") schalenförmig auf einem im Allgemeinen unter den Reaktionsbedingungen inerten Trägermaterial, wie Quarz (SiO₂), Porzellan, Magnesiumoxid, Zinndioxid, Siliciumcarbid, Rutil, Tonerde (Al₂O₃), Aluminiumsilikat, Magnesiumsilikat (Steatit), Zirkoniumsilikat oder Cersilikat oder Mischungen dieser Trägermaterialien aufgebracht ist. Als katalytisch aktiver Bestandteil der katalytisch aktiven Masse dieser Schalenkatalysatoren dient im Allgemeinen neben Titandioxid in Form seiner Anatasmodifikation Vanadiumpentoxid. Des weiteren kann in der katalytisch aktiven Masse in geringen Mengen eine Vielzahl anderer oxidischer Verbindungen enthalten sein, die als Promotoren die Aktivität und Selektivität des Katalysators beeinflussen, indem sie seine Aktivität absenken oder erhöhen. Als solche Promotoren seien beispielhaft die Alkalimetalloxide, insbesondere Lithium-, Kalium-, Rubidium- und Cäsiumoxid, Thallitirn-(I)-oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid und Phosphorpentoxid genannt. Als die Aktivität vermindernde und die Selektivität erhöhende Promotoren wirken z.B. die Alkalimetalloxide, wohingegen oxidische Phosphorverbindungen, insbesondere Phosphorpentoxid, die Aktivität des Katalysators erhöhen, aber dessen Selektivität vermindern.

Zur Herstellung derartiger Schalenkatalysatoren wird nach den Verfahren von DE-A 1642938 und DE-A 1769998 eine wäßrige und/oder ein organisches Lösungsmittel enthaltende Lösung oder Suspension der Aktivmassenbestandteile und/oder deren Vorläuferverbindungen, welche im folgenden als "Maische" bezeichnet wird, auf das Trägermaterial in einer beheizten Dragiertrommel bei erhöhter Temperatur aufgesprüht, bis der gewünschte Aktivmassenanteil am Katalysatorgesamtgewicht erreicht ist. Nach DE 2106796 läßt sich die Beschichtung auch in Wirbelbeschichtern durchführen, wie sie z.B. in DE 1280756 angegeben sind. Beim Aufsprühen in der Dragiertrommel sowie bei Beschichten im Wirbelbett treten allerdings hohe Verluste auf, da erhebliche Mengen der Maische vernebelt bzw. durch Abrasion Teile der bereits aufbeschichteten Aktivmasse wieder abgerieben und durch das Abgas ausgetragen werden. Der Aktivmasseanteil am Gesamtkatalysator soll im Allgemeinen nur eine geringe Abweichung vom Sollwert haben soll, da durch die Menge der aufgebrachten Aktivmasse und damit die Schichtdicke der Schale Aktivität und Selektivität des Katalysators stark beeinflußt werden. Daher muß der Katalysator bei den geschilderten Herstellungsweisen häufig zur Bestimmung der aufgebrachten Aktivmassemenge abgekühlt, aus der Dragiertrommel bzw. der Wirbelschicht entnommen und nachgewogen werden. Wird zuviel Aktivmasse auf dem Katalysatorträger abgeschieden, ist im Allgemeinen eine nachträgliche, schonende Entfernung der zuviel aufgetragenen Aktivmassemenge ohne Beeinträchtigung der Festigkeit der Schale, insbesondere ohne Rißbildung in der Katalysatorschale, nicht möglich.

Um dieses Problem abzumildern wurde auch in der Technik dazu übergegangen, der Maische organische Binder, bevorzugt Copolymere von Vinylacetat/Vinyllaurat, Vinylacetat/Acrylat, Styrol/Acrylat, Vinylacetat/Maleat sowie Vinylacetat/Ethylen, vorteilhaft in Form einer wäßrigen Dispersion, zuzusetzen, wobei Bindermengen von 10 - 20 Gew.-%, bezogen auf den Feststoffgehalt der Maische, eingesetzt wurden (EP-A 744214). Wird die Maische ohne organische Bindemittel auf den Träger aufgetragen, so sind Beschichtungstemperaturen über 150°C von Vorteil. Bei Zusatz der oben angegebenen Bindemittel liegen die brauchbaren Beschichtungstemperaturen je nach verwendetem Bindemittel zwischen 50 und 450°C (DE 2106796). Die aufgetragenen Bindemittel brennen nach dem Einfüllen des Katalysators in den Reaktor und Inbetriebnahme des Reaktors innerhalb kurzer Zeit aus. Der Binderzusatz hat zudem den Vorteil, dass die Aktivmasse gut auf dem Träger haftet, so dass Transport und Einfüllen des Katalysators erleichtert werden.

Weitere geeignete Verfahren zur Herstellung von Schalenkatalysatoren für die katalytische Gasphasenoxidation von aromatischen Kohlenwasserstoffen zu Carbonsäuren und/oder Carbonsäureanhydriden sind in WO-98/37967 bzw. EP-A 714700 beschrieben. Die katalytisch aktive Metalloxide enthaltende Schicht wird in der Form auf ein Trägermaterial aufgebracht, dass aus einer Lösung und/oder einer Suspension der katalytisch aktiven Metalloxide und/oder deren Vorläuferverbindungen, gegebenenfalls in Anwesenheit von Hilfsmitteln für die Katalysatorherstellung, zunächst ein Pulver hergestellt wird, das anschließend für die Katalysatorherstellung auf dem Träger, gegebenenfalls nach vorausgegangener Konditionierung sowie gegebenenfalls nach Wärmebehandlung zur Erzeugung der katalytisch aktiven Metalloxide schalenförmig aufgebracht und der auf diese Weise beschichtete Träger einer Wärmebehandlung zur Erzeugung der katalytisch aktiven Metalloxide oder einer Behandlung zur Entfernung flüchtiger Bestandteile unterzogen wird.

Zur Durchführung der Gasphasenoxidation werden die Katalysatoren zunächst in die Reaktionsrohre des Reaktors, die von außen auf die Reaktionstemperatur, beispielsweise mittels Salzschmelzen, thermostatisiert sind, gefüllt. Über die so bereitete Katalysatorschüttung wird das Reaktionsgas geleitet.

Das dem Katalysator zugeführte Reaktionsgas wird im Allgemeinen durch Vermischen von einem molekularen Sauerstoff enthaltend Gas, das außer Sauerstoff noch geeignete Reaktionsmoderatoren und/oder Verdünnungsmittel, wie Dampf, Kohlendioxid und/oder Stickstoff, enthalten kann, mit dem zu oxidierenden aromatischen Kohlenwasserstoff erzeugt, wobei das den molekularen Sauerstoff enthaltende Gas im Allgemeinen 1 bis 100, vorzugsweise 2 bis 50 und besonders bevorzugt 10 bis 30 mol-% Sauerstoff, 0 bis 30 mol-%, vorzugsweise 0 bis 10 mol-% Wasserdampf sowie 0 bis 50 mol-%, vorzugsweise 0 bis 1 mol-% Kohlendioxid, Rest Stickstoff, enthalten kann. Zur Erzeugung des Reaktionsgases wird das den molekularen Sauerstoff enthaltende Gas im Allgemeinen mit 30 bis 150 g je Nm³ Gas des zu oxidierenden, aromatischen Kohlenwasserstoffs beschickt.

Das zu oxydierende o-Xylol/Naphthalingemisch kann in einem breiten Mischungsverhältnis beispielsweise im Gewichtsverhältnis 1:99 bis 99:1 eingesetzt werden, insbesondere jedoch in einem solchen Verhältnis, dass der o-Xylol-Gehalt mindestens 30 , vorzugsweise mindestens 50 und insbesondere mindestens 70 Gew.-% beträgt. Das Verhältnis o-Xylol/Naphthalin kann über die Katalysatorlebensdauer konstant bleiben oder auch verändert werden. So kann z.B. vorteilhaft im Laufe der Zeit der Gehalt von o-Xylol erhöht werden und dadurch eine höhere Ausbeute erzielt werden.

Die Umsetzung wird bei Temperaturen von im Allgemeinen 300 bis 450, vorzugsweise von 320 bis 420 und besonders bevorzugt von 340 bis 400°C und bei einem Überdruck von im Allgemeinen 0,1 bis 2,5, vorzugsweise von 0,3 bis 1,5 bar mit einer Raumgeschwindigkeit von Allgemeinen 750 bis 500 h⁻¹ durchgeführt.

Die Herstellung von Phthalsäureanhydrid über den erfindungsgemäß zu verwendenden geschichteten Katalysatoren erfolgt im übrigen in an sich bekannter Weise, wie dies z.B. durch K. Towae, W. Enke, R. Jäckh u. N. Bhargawa in "Phthalic Acid and Derivatives", Ullmann's Encylopedia of Industrial Chemistry, Vol. A 20, 1992, 181 Einzelnen beschrieben ist.

### Beispiele

### Beispiel 1: Herstellung des Katalysators I

700g Steatit (Magnesiumsilikat) in Form von Ringen mit einem äußeren Durchmesser von 8 mm, einer Länge von 6 mm und einer Wandstärke von 1,5 mm wurden in einer Dragiertrommel auf 210°C erhitzt und mit einer Suspension aus 400,0g Anatas mit einer BET-Oberfläche von 21m²/g, 57,6g Vanadyloxalat, 14,4g Antimontrioxid, 2,5g Ammoniumhydrogenphosphat, 2,60g Cäsiumsulfat, 618g Wasser und 128g Formamid besprüht, bis das Gewicht der aufgetragenen Schicht 10,5 % des Gesamtgewichts des fertigen Katalysators betrug. Die auf diese Weise aufgebrachte katalytisch aktive Masse, also die Katalysatorschale, bestand aus 0,15 Gew.-% Phosphor (berechnet als P), 7,5 Gew.-% Vanadium (berechnet als V₂O₅), 3,2 Gew.-% Antimon (berechnet als Sb₂O₃), 0,4 Gew.-% Cäsium (berechnet als Cs) und 88,75 Gew.-% Titandioxid.

### Beispiel 2: Herstellung des Katalysators II

700g Steatit (Magnesiumsilikat) Ringe mit einem äußeren Durchmesser von 8 mm, einer Länge von 6 mm und einer Wandstärke von 1,5 mm wurden in einer Dragiertrommel auf 210°C erhitzt und mit einer Suspension aus 400.0g Anatas mit einer BET-Oberflache von 20m²/g, 57,6g Vanadyloxalat, 14,4g Antimontrioxid, 2,5g Ammoniumhydrogenphosphat, 0,32g Cäsiumsulfat, 618g Wasser und 128g Formamid besprüht, bis das Gewicht der aufgetragenen Schicht 10,5 % des Gesamtgewichts des fertigen Katalysators betrug. Die auf diese Weise aufgebrachte katalytisch aktive Masse, also die Katalysatorschale, bestand aus 0,15 Gew.-% Phosphor (berechnet als P), 7,5 Gew.-% Vanadium (berechnet als V₂O₅), 3,2 Gew.-% Antimon (berechnet als Sb₂O₃), 0,05 Gew.-% Cäsium (berechnet als Cs) und 89,1 Gew.-% Titandioxid.

### Beispiel 3: Herstellung eines Vergleichskatalysators

700g Steatit (Magnesiumsilikat) in Form von Ringen mit einem äußeren Durchmesser von 8 mm, einer Länge von 6 mm und einer Wandstärke von 1,5 mm wurden in einer Dragiertrommel auf 210°C erhitzt und mit einer Suspension aus 400,0g Anatas mit einer BET-Oberfläche von 20 m²/g, 57,6g Vanadyloxalat, 14.4g Antimontrioxid, 2,5g Ammoniumhydrogenphosphat, 0,65g Cäsiumsulfat, 618g Wasser und 128g Formamit besprüht, bis das Gewicht der aufgetragenen Schicht 10,5 % des Gesamtgewichts des fertigen Katalysators betrug. Die auf diese Weise aufgebrachte katalytisch aktive Masse, also die Katalysatorschale, bestand aus 0,15 Gew.-% Phosphor (berechnet als P), 7,5 Gew.-% Vanadium (berechnet als V₂O₅), 3,2 Gew.-% Antimon (berechnet als Sb₂O₃), 0,1 Gew.-% Cäsium (berechnet als Cs) und 89,05 Gew.-% Titandioxid.

### Beispiel 4: Herstellung von PSA

Man füllte jeweils 1,30 m des Katalysators II bzw. des Vergleichskatalysators aus Beispiel 3 und anschließend 1,60 m des Katalysators I in ein 3,85 m langes Eisenrohr mit einer lichten Weite von 25 mm. Das Eisenrohr war zur Temperaturregelung von einer Salzschmelze umgeben; eine 4 mm Thermohülse mit eingebautem Zugelement diente der Katalysatortemperaturmessung. Durch das Rohr wurde stündlich von oben nach unten 4,0 Nm³-Luft mit Beladungen an einem Gemisch von o-Xylol/Naphthalin von 75 Gew.-% : 25 Gew.-% von 0 bis ansteigend etwa 80 g/Nm³-Luft geleitet. Es wurde wie folgt weiterverfahren bzw. es wurden die folgenden Ergebnisse erhalten:
a) Der Längenanteil von Katalysator II an der Länge der gesamten Katalysatorschüttung war 45%. Der Gehalt an Cäsium in Katalysator II bezogen auf den in Katalysator I war 12,5 Gew.-%. Weitere Versuchsparameter und Ergebnisse:

| Beladung [g Gemisch/ Nm³ Luft] | Bad-temperatur [°C] | PSA-Ausbeute im Durchschnitt | Phthalid im Roh-PSA [Gew.-%] | Naphthochinon im Roh-PSA [Gew.-%] |
|---|---|---|---|---|
| 65-80 | 357-354 | 110 | <0,2 | <0,15 |

b) Der Längenanteil des Vergleichskatalysators an der Länge der gesamten Katalysatorschüttung war 45%. Der Gehalt an Cäsium im Vergleichskatalysator bezogen auf den in Katalysator I war 25 Gew.-%. Weitere Versuchsparameter und Ergebnisse:

| Beladung [g Gemisch/ Nm³ Luft] | Bad-temperatur [°C] | PSA-Ausbeute im Durchschnitt [Gew.-%] | Phthalid im Roh-PSA [Gew.-%] | Naphthochinon im Roh-PSA [Gew.-%] |
|---|---|---|---|---|
| >65 | <357 | * | >0,2 | >0,2 |

| | | | | |
|---|---|---|---|---|
| *nicht fahrbar, da Nebenprodukte zu hoch | | | | |

## Patentansprüche

1. Verfahren zur Herstellung von Phthalsäureanhydrid durch katalytische Gäsphasenoxidation von o-Xylol/Naphthalingemischen mit molekularem Sauerstoff unter Verwendung eines
Katalysators I in einer ersten Schicht auf der Gaseintrittsseite, die 25 bis 75 Volumenprozent des gesamten Katalysatorvolumens ausmacht, enthaltend, jeweils bezogen auf die katalytisch wirksame Masse, 1 bis 10 Gew.-% Vanadiumoxid (berechnet als V₂O₅), 1 bis 10 Gew.-% Antimonoxid (berechnet als Sb₂O₃) und 80 bis 98 Gew.-% Titandioxid vom Anatas-Typ mit einer BET-Oberfläche von 13 bis 28 m²/g sowie 0,05 bis 1 Gew.-% Cäsium (berechnet als Cs), aufgebracht auf einem Steatitträger und eines
Katalysators II in einer zweiten Schicht, die die restlichen 75 bis 25 Volumenprozent des gesamten Katalysatorvolumens ausmacht, enthaltend, jeweils bezogen auf die katalytisch wirksame Masse, 1 bis 10 Gew.-% Vanadiumoxid (berechnet als V₂O₅), 1 bis 10 Gew.-% Antimonoxid (berechnet als Sb₂O₃) und 80 bis 98 Gew.-% Titanoxid vom Anatas-Typ mit einer BET-Oberfläche von 13 bis 28 m²/g und 0,01 bis 1 Gew.-% Phosphoroxid (berechnet als P) sowie 0.01 bis 0,2 Gew.-% Cäsium (berechnet als Cs), aufgebracht auf einem Steatitträger,
**dadurch gekennzeichnet, dass** der Cäsiumgehalt des Katalysators II weniger als 15 Gew.-% des Cäsiumgehalts des Katalysators I beträgt und dass der Katalysator I und der Katalysator II ohne Zusatz von Verbindungen des Niobs hergestellt sind.

2. Verfahren nach Anspruch 1, **dadurch gekennzeichnet, dass** der Katalysator I auf den ersten 40 bis 60 Volumenprozent des Katalysatorvolumens in Strömungsrichtung des Gemisches aus o-Xylol-/Naphthalinmischungen und Reaktionsgas eingesetzt wird und der Katalysator II auf den letzten 60 bis 40 Volumenprozent des Katalysatorvolumens.

3. Verfahren nach Anspruch 1, **dadurch gekennzeichnet, dass** Titandioxid vom Anatas-Typ mit einer BET-Oberfläche von 19 bis 21 m²/g verwendet wird.

4. Verfahren nach Anspruch 1, **dadurch gekennzeichnet, dass** die Zusammensetzung der Katalysatoren I und II bis auf den Cäsiumgehalt identisch ist.

5. Verfahren nach Anspruch 1, **dadurch gekennzeichnet, dass** der Cäsium-Gehalt des Katalysators II 10 bis 13 Gew.-% des Cäsiumgehalts des Katalysators I beträgt.

6. Verfahren nach Anspruch 1, **dadurch gekennzeichnet, dass** der o-Xylol-Gehalt im Gemisch der Ausgangsstoffe o-Xylol und Naphthalin mindestens 30 Gew.-% beträgt.

7. Verfahren nach Anspruch 1, **dadurch gekennzeichnet, dass** der o-Xylol-Gehalt im Gemisch der Ausgangsstoffe o-Xylol und Naphthalin mindestens 50 Gew.-% beträgt.

8. Verfahren nach Anspruch 1, **dadurch gekennzeichnet, dass** der o-Xylol-Gehalt im Gemisch der Ausgangsstoffe o-Xylol und Naphthalin mindestens 70 Gew.-% beträgt.

## Claims

1. A process for preparing phthalic anhydride by catalytic gas-phase oxidation of o-xylene/naphthalene mixtures by molecular oxygen using
a catalyst I in a first zone on the gas inlet side which makes up from 25 to 75 percent by volume of the total catalyst volume, comprising, in each case based on the catalytically active composition, from 1 to 10% by weight of vanadium oxide (calculated as V₂O₅), from 1 to 10% by weight of antimony oxide (calculated as Sb₂O₃) and from 80 to 98% by weight of titanium dioxide of the anatase type having a BET surface area of from 13 to 28 m²/g and also from 0.05 to 1% by weight of cesium (calculated as Cs) applied to a steatite support and
a catalyst II in a second zone which makes up the remaining 75 to 25 percent by volume of the total catalyst volume, comprising, in each case based on the catalytically active composition, from 1 to 10% by weight of vanadium oxide (calculated as V₂O₅), from 1 to 10% by weight of antimony oxide (calculated as Sb₂O₃) and from 80 to 98% by weight of titanium oxide of the anatase type having a BET surface area of from 13 to 28 m²/g and from 0.01 to 1% by weight of phosphorus oxide (calculated as P) and also from 0.01 to 0.2% by weight of cesium (calculated as Cs) applied to a steatite support,
wherein the cesium content of the catalyst II is less than 15% by weight of the cesium content of the catalyst I and the catalyst I and the catalyst II have been prepared without addition of compounds of niobium.

2. A process as claimed in claim 1, wherein the catalyst I is used in the first 40 to 60 percent by volume of the catalyst volume in the flow direction of the mixture of o-xylene/naphthalene mixture and reaction gas and the catalyst II is used in the last 60 to 40 percent by volume of the catalyst volume.

3. A process as claimed in claim 1, wherein titanium dioxide of the anatase type having a BET surface area of from 19 to 21 m²/g is used.

4. A process as claimed in claim 1, wherein the composition of the catalysts I and II is identical except for the cesium content.

5. A process as claimed in claim 1, wherein the cesium content of the catalyst II is from 10 to 13% by weight of the cesium content of the catalyst I.

6. A process as claimed in claim 1, wherein the o-xylene content of the mixture of the starting substances of o-xylene and naphthalene is at least 30% by weight.

7. A process as claimed in claim 1, wherein the o-xylene content of the mixture of the starting substances of o-xylene and naphthalene is at least 50% by weight.

8. A process as claimed in claim 1, wherein the o-xylene content of the mixture of the starting substances of o-xylene and naphthalene is at least 70% by weight.

## Revendications

1. Procédé de production d'anhydride phtalique par oxydation catalytique en phase gazeuse de mélanges o-xylène/naphtalène, à l'aide de l'oxygène moléculaire, en mettant en oeuvre,
un catalyseur (I), dans une première couche située sur le côté d'entrée du gaz et représentant 25% à 75% en volume par rapport au volume total de catalyseur, et contenant, à chaque fois par rapport à la masse à activité catalytique, de l'oxyde de vanadium (calculé comme V₂O₅) à raison de 1 % à 10% en poids, de l'oxyde d'antimoine (calculé comme Sb₂O₃) à raison de 1% à 10% en poids, et du dioxyde de titane de type anatase présentant une surface BET comprise entre 13 m²/g à 28 m²/g, à raison de 80% à 98% en poids, ainsi que du césium (calculé comme Cs) à raison de 0,05% à 1% en poids, appliquée sur un support de stéatite, et
un catalyseur (II), dans une deuxième couche représentant les 75% à 25% en volume restant, par rapport au volume total de catalyseur, et contenant, à chaque fois par rapport à la masse à activité catalytique, de l'oxyde de vanadium (calculé comme V₂O₅) à raison de 1% à 10% en poids, de l'oxyde d'antimoine (calculé comme Sb₂O₃) à raison de 1% à 10% en poids, et de l'oxyde de titane de type anatase présentant une surface BET comprise entre 13 m²/g à 28 m²/g, à raison de 80% à 98% en poids, et de l'oxyde de phosphore (calculé comme P) à raison de 0,01% à 1% en poids, ainsi que du césium (calculé comme Cs) à raison de 0,01% à 0,2% en poids, appliquée sur un support de stéatite,
**caractérisé en ce que** la teneur en césium du catalyseur (II) est inférieur à 15% en poids par rapport à la teneur en césium du catalyseur (I), et **caractérisé en ce que** l'on prépare le catalyseur (I) et le catalyseur (II) sans ajout de composés de niobium.

2. Procédé selon la revendication 1, **caractérisé en ce que** l'on met en oeuvre le catalyseur (I), sur les 40 à 60 premiers % en volume par rapport au volume de catalyseur, dans le sens d'écoulement du mélange constitué de mélanges o-xylène/naphtalène et de gaz de réaction, et le catalyseur (II), sur les 60 à 40 derniers % en volume par rapport au volume de catalyseur.

3. Procédé selon la revendication 1, **caractérisé en ce que** l'on met en oeuvre du dioxyde de titane du type anatase présentant une surface BET comprise entre 19 m²/g et 21 m²/g.

4. Procédé selon la revendication 1, **caractérisé en ce que** la composition des catalyseurs (I) et (II) est identique à l'exception de la teneur en césium.

5. Procédé selon la revendication 1, **caractérisé en ce que** la teneur en césium du catalyseur (II) est de 10% à 13% en poids de la teneur en césium du catalyseur (I).

6. Procédé selon la revendication 1, **caractérisé en ce que** la teneur en o-xylène du mélange des matières de départ constitué de o-xylène et de naphtalène, est d'au moins de 30% en poids.

7. Procédé selon la revendication 1, **caractérisé en ce que** la teneur en o-xylène du mélange des matières de départ constitué de o-xylène et de naphtalène, est d'au moins de 50% en poids.

8. Procédé selon la revendication 1, **caractérisé en ce que** la teneur en o-xylène du mélange des matières de départ constitué de o-xylène et de naphtalène, est d'au moins de 70% en poids.
